# FASCICULE DE BREVET EUROPEEN

(11) **EP 2 852 761 B1**
(45) Date de publication et mention de la délivrance du brevet: **06.04.2016**
(21) Numéro de dépôt: 13730290.7
(22) Date de dépôt: 21.05.2013
(51) Int. Cl.: F04B 43/12, F04B 43/00, F04B 43/08, F04B 49/06, A61M 5/142

(54) **POMPE PERISTALTIQUE LINEAIRE**
LINEARE PERISTALTISCHE PUMPE
LINEAR PERISTALTIC PUMP

(30) Priorité: 23.05.2012 FR 1254691
(43) Date de publication de la demande: 01.04.2015
(73) Titulaire: Physidia, 49124 Saint-Barthélémy-d'Anjou (FR)
(72) Inventeur: FERME, Philippe, F-49124 Le Plessis Grammoire (FR); MARINE, Julien, F-49000 Angers (FR); VINCENT, Eric, F-49000 Angers (FR)
(74) Mandataire: Dutreix, Hugues Ours
(86) Numéro de dépôt international: PCT/FR2013/051100
(87) Numéro de publication internationale: WO 2013/175115

(56) Documents cités:
- EP-A1- 0 837 242
- BE-A- 685 301
- FR-A1- 2 594 496
- GB-A- 953 579
- GB-A- 2 230 301
- US-A- 4 617 014
- US-A1- 2006 228 240
- US-A1- 2010 106 082

## Description

La présente invention concerne de manière générale les pompes péristaltiques.

L'invention concerne plus particulièrement une pompe péristaltique, par exemple pour machine de dialyse, comprenant, d'une part, un plateau, appelé corps de pompe, qui comprend une surface sensiblement plane contre laquelle est destiné à être positionné un tube souple de passage de fluide, et, d'autre part, un système d'application d'effort comprenant une pluralité d'organes d'appui, tels que des galets, et des moyens d'entrainement en déplacement desdits organes d'appui permettant de déplacer lesdits organes d'appui contre le tube pour le déformer contre ledit corps de pompe.

On constate qu'avec certaines pompes péristaltiques, le tube souple peut ne pas être suffisamment serré entre le corps de pompe et le système d'application d'effort, ce qui ne permet pas de faire circuler le fluide à travers le tube avec un débit et/ou une régularité suffisante. Inversement, avec d'autres pompes, le tube souple se retrouve serré de manière trop importante entre le corps de pompe et le système d'application d'effort, ce qui peut aussi gêner la circulation du fluide et dégrader le tube.

On connait aussi des documents GB 2.230.301, GB 953.579, BE 685.301 et EP 0.837.242, des pompes comprenant une partie support de tube de passage de fluide déplaçable par rapport à un système d'application d'effort. Le déplacement autorisé entre la partie support de tube de passage de fluide et le système d'application d'effort sert à libérer un passage pour l'introduction du tube ou pour diminuer ou augmenter la surface de contact entre le système d'application d'effort et le tube afin de réduire ou d'augmenter le débit de fluide à travers le tube. Cependant, le positionnement relatif entre le corps de pompe et le système d'application d'effort reste difficile à réaliser de manière fiable, précise et répétable. En outre, de telles pompes n'empêchent pas que le positionnement relatif entre le corps de pompe et le système d'application ne soit pas adapté au tube, entrainant ainsi un risque de dégradation du tube. Le document FR 2.594.496 concerne un conduit de transfert de fluides présentant une partie intermédiaire comportant une section plus large et une paroi d'épaisseur notablement plus faible et une résistance sensiblement plus faible à la déformation, que celle des autres parties dudit conduit.

La présente invention a pour but de proposer une pompe péristaltique permettant de résoudre les problèmes énoncés ci-dessus.

A cet effet, l'invention a pour objet une pompe péristaltique, par exemple pour machine de dialyse, comprenant, d'une part, un plateau, appelé corps de pompe, qui comprend une surface sensiblement plane contre laquelle est destiné à être positionné un tube souple de passage de fluide, et, d'autre part, un système d'application d'effort comprenant une pluralité d'organes d'appui, tels que des galets, et des moyens d'entrainement en déplacement desdits organes d'appui permettant de déplacer lesdits organes d'appui contre le tube pour le déformer contre ledit corps de pompe, ledit corps de pompe étant monté mobile par rapport au système d'application d'effort entre une position écartée dudit système d'application d'effort, et une position rapprochée dudit système d'application d'effort, caractérisée en ce que ladite pompe comprend des moyens de détermination d'une grandeur représentative de l'effort appliqué sur le tube, à l'état positionné dudit tube entre le système d'application d'effort et le corps de pompe, et en ce que ladite pompe comprend aussi des moyens de pilotage du déplacement du corps de pompe par rapport au système en fonction de ladite grandeur déterminée.

Ledit effort déterminé correspond à l'effort de serrage que subit le tube pris entre le système d'application d'effort et ledit corps de pompe au cours du rapprochement de l'un vers l'autre. La prise en compte de cet effort appliqué sur le tube pour piloter le déplacement du corps de pompe relativement au système d'application d'effort, permet au corps de pompe de passer d'une position écartée du système d'application d'effort dans laquelle le tube souple peut être positionné librement afin de l'introduire aisément dans la pompe, à une position rapprochée dans laquelle le tube est maintenu serré avec un effort de serrage adapté automatiquement grâce à la mesure de l'effort appliqué au tube, pour que ledit effort de serrage soit suffisant afin de permettre au système d'application d'effort de le déformer, et ainsi de faire circuler efficacement le fluide, tout en étant limité pour ne pas dégrader le tube. Ledit effort mesuré est principalement transversal, de préférence orthogonal, à l'axe du tube. Avantageusement, cette mesure d'effort est réalisée en l'absence de circulation de fluide à travers le conduit, c'est-à-dire à l'arrêt (les uns par rapport aux autres) des éléments, par exemple des rouleaux, du système d'application d'effort qui servent à faire circuler le fluide.

Ledit effort est mesuré à une fréquence donnée jusqu'à atteindre la valeur de consigne, ou une valeur contenue dans une plage donnée autour de la valeur de consigne, pour pouvoir piloter le déplacement relatif du corps de pompe par rapport au système d'application d'effort sensiblement en temps réel.

Le choix de la mesure de l'effort de serrage pour piloter le déplacement du corps de pompe par rapport au système d'application d'effort est particulièrement intéressant pour s'assurer d'un débit de fluide suffisant à travers le tube, tout en réduisant le risque de dégradation du tube, puisque grâce à ce paramètre d'effort appliqué, un même serrage peut être précisément, de manière fiable et répétable, obtenu pour différent diamètre ou matière de tube.

En particulier, le pilotage du déplacement du corps de pompe en fonction de l'effort de serrage appliqué au tube permet d'adapter automatiquement l'effort de serrage du tube pour atteindre une valeur de consigne afin que ledit effort soit suffisant pour permettre au système d'application d'effort de déformer le tube, et ainsi de faire circuler efficacement le fluide, tout en étant limité pour ne pas dégrader le tube et permettre une circulation efficace du fluide.

Une telle conception de la pompe facilite la mise en place du tube, par exemple une ligne à sang à usage unique, dans le corps de pompe.

Selon une caractéristique avantageuse de l'invention, ledit corps de pompe est monté mobile en translation suivant une direction transversale, de préférence orthogonale, à ladite surface plane du corps de pompe.

Selon une caractéristique avantageuse de l'invention, ladite pompe comprend des moyens moteurs d'entrainement en déplacement dudit corps de pompe entre ladite position rapprochée et ladite position écartée.

Selon une caractéristique avantageuse de l'invention, lesdits moyens de pilotage comprennent des moyens de définition d'une valeur de consigne de ladite grandeur, et des moyens de régulation configurés pour réguler le déplacement du corps de pompe dans le sens d'un rapprochement ou d'un écartement du système d'application d'effort en fonction de la valeur déterminée de ladite grandeur pour atteindre ladite valeur de consigne.

Selon une caractéristique avantageuse de l'invention, lesdits moyens de détermination d'une grandeur représentative de l'effort appliqué sur le tube, comprennent au moins une jauge de contrainte, de préférence une pluralité de jauges de contrainte.

Selon une caractéristique avantageuse de l'invention, lesdits moyens d'entrainement en déplacement des organes d'appui comprennent un élément en boucle qui relie entre eux les organes d'appui, et deux rouleaux rotatifs, positionnés à l'intérieur et aux extrémités opposées dudit élément en boucle, au moins l'un des rouleaux étant motorisé pour entrainer en déplacement l'élément en boucle autour desdits rouleaux.

Selon une caractéristique avantageuse de l'invention, ladite pompe comprend un bâti dans lequel sont logés le système et le corps de pompe, et le corps de pompe est monté mobile par rapport audit bâti.

Selon une caractéristique avantageuse de l'invention, ladite pompe comprend un bâti dans lequel sont logés le système et le corps de pompe et ladite pompe comprend une membrane qui, à l'état inséré du tube entre le système d'application d'effort et le corps de pompe, s'étend autour de la paroi périphérique dudit tube pour former en coopération avec une paroi du bâti une enveloppe de protection autour dudit tube.

La membrane souple étanche permet d'isoler l'intérieur de la pompe vis-à-vis du tube, ce qui permet d'éviter de souiller l'intérieur de la machine en cas de fuite. En outre, la membrane souple étanche améliore aussi l'isolation phonique de la pompe.

Selon une caractéristique avantageuse de l'invention, ladite membrane est réalisée en un matériau souple étanche aux liquides.

L'invention sera bien comprise à la lecture de la description suivante d'exemples de réalisation, en référence aux dessins annexés dans lesquels :

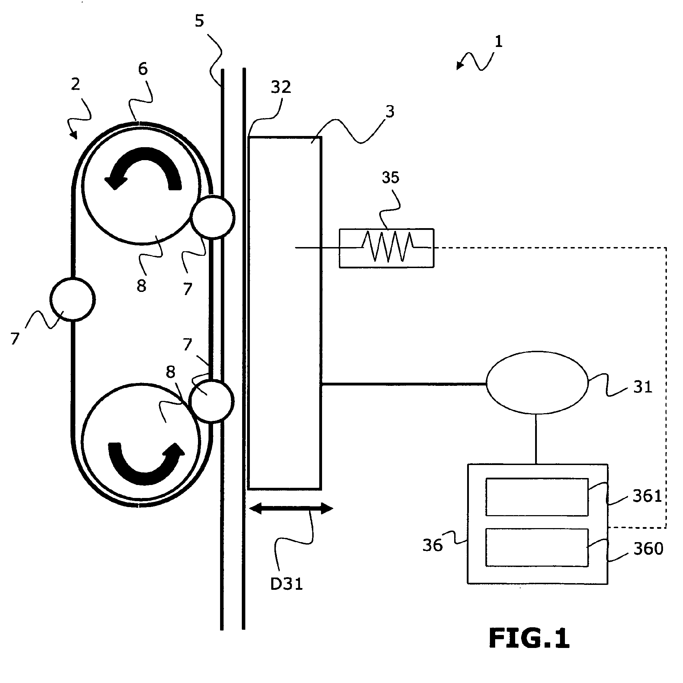

- la figure 1 est une vue schématique d'une pompe péristaltique linéaire selon un mode de réalisation de l'invention ;

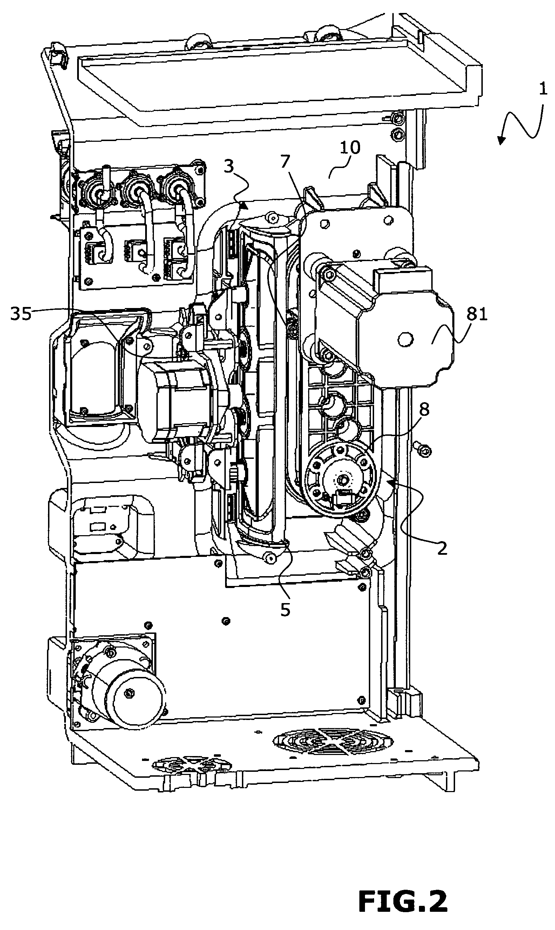

- la figure 2 est une vue en perspective de l'intérieur d'une partie de la pompe de la figure 1 ;
- la figure 3 est une vue en perspective d'une membrane étanche destinée à entourer le tube introduit dans la pompe de la figure 1 ;
- la figure 4 est une vue en perspective du corps de pompe et du système d'application d'effort de la pompe de la figure 1 en position écartée l'un de l'autre pour l'insertion dudit tube ;
- la figure 5 est une vue en perspective du corps de pompe et du système d'application d'effort de la pompe de la figure 1 en position rapprochée l'un de l'autre.

En référence aux figures et comme rappelé ci-dessus, l'invention concerne une pompe 1 péristaltique linéaire pour machine de dialyse. Ladite pompe 1 péristaltique comporte un plateau, appelé corps de pompe 3, qui comprend une surface sensiblement plane 32 contre laquelle est destiné à être positionné un tube souple 5 de passage de fluide. Ladite surface 32 est considérée comme plane, ou plate, c'est-à-dire droite, par opposition aux surfaces de contact avec le tube des corps de pompes péristaltiques dites rotatives qui présentent une forme en arc de cercle.

La pompe 1 péristaltique comporte aussi un système 2 d'application d'effort muni d'une pluralité d'organes d'appui 7 et des moyens d'entrainement en déplacement desdits d'organes d'appui 7 permettant de déplacer lesdits organes d'appui contre le tube pour le déformer contre ledit corps de pompe 3.

Ledit corps de pompe 3 et ledit système 2 sont agencés l'un par rapport à l'autre pour permettre le positionnement d'un tube souple 5 de passage de fluide, entre la surface plane 32 du corps de pompe 3 et le système 2. Le déplacement desdits d'organes d'appui 7 s'effectue suivant un chemin en boucle fermée comme détaillé ci-après. Ce chemin en boucle fermée comprend une portion orientée du côté du corps de pompe 3 qui permet aux organes d'appui 7 parcourant cette portion de presser le conduit contre la surface plane 32 du corps de pompe 3.

Préférentiellement, ladite pompe est utilisée en tant que pompe à sang d'une machine de dialyse. Dans ce cas, ledit tube 5 forme une ligne de sang.

Ladite surface plane 32 est orientée vers ledit système 2 d'application d'effort pour permettre à ce dernier d'appliquer un effort de pression sur la paroi périphérique dudit tube souple 5 positionné contre le corps de pompe 3.

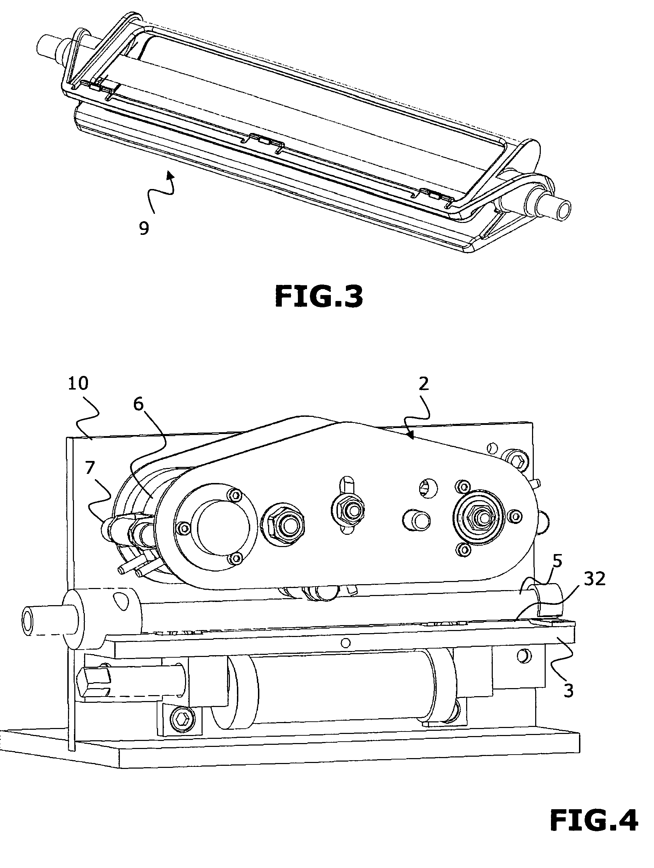

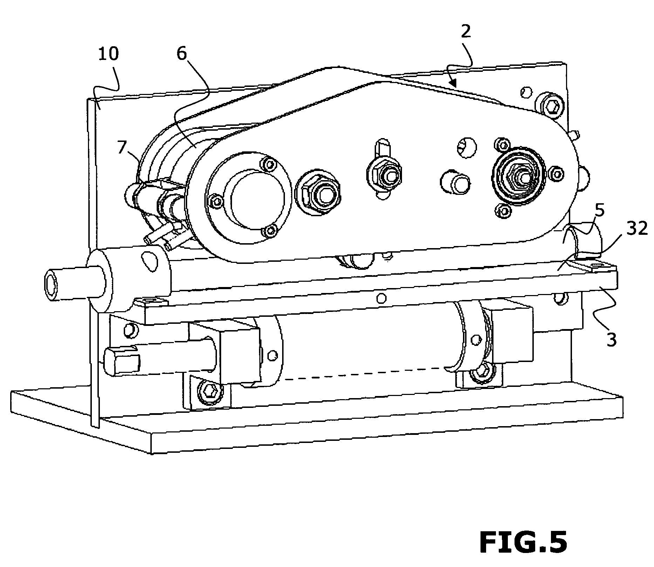

Ledit corps de pompe 3 est monté mobile par rapport au système 2 d'application d'effort entre une position écartée dudit système 2 d'application d'effort (voir figure 4), et une position rapprochée dudit système 2 d'application d'effort (voir figure 5). Ledit corps de pompe 3 et le système 2 d'application définissent ainsi entre eux un espace d'insertion du tube souple sensiblement droit puisqu'il s'étend le long de la surface plane 32 du corps de pompe.

En position écartée dudit système 2 d'application d'effort par rapport au corps de pompe, l'espace laissé libre entre le système 2 d'application d'effort et le corps de pompe est suffisant pour introduire librement le tube 5 entre le système 2 d'application d'effort et le corps de pompe 3. Une fois le tube 5 positionné contre la face plane 32 du corps de pompe 3, le corps de pompe 3 et le système 2 sont amenés en position rapprochée pour permettre au système 2 d'appliquer un effort sur la paroi périphérique du tube 5 depuis une extrémité de l'espace d'insertion du tube vers l'autre extrémité.

L'application d'un effort sur la paroi périphérique du tube 5 est réalisée par appui desdits organes d'appui 7 sur la paroi périphérique dudit tube pour la déformer progressivement le long de sa portion en appui sur la face plane 32 du corps de pompe 3 et ainsi faire circuler le fluide contenu dans le tube 5 depuis une extrémité de la surface 32 vers son autre extrémité. Ledit corps de pompe 3 est monté mobile en translation suivant une direction D31 transversale, de préférence orthogonale, à ladite surface plane 32 du corps de pompe 3.

Ladite pompe 1 comprend des moyens moteurs 31 d'entrainement en déplacement dudit corps de pompe 3 entre ladite position rapprochée et ladite position écartée.

Ladite pompe 1 comprend aussi des moyens 35 de détermination d'une grandeur représentative de l'effort appliqué sur la paroi périphérique du tube 5, à l'état positionné dudit tube 5 entre le système d'application d'effort 2 et le corps de pompe 3. Lesdits moyens 35 de détermination d'une grandeur représentative de l'effort appliqué sur le tube 5, comprennent au moins une jauge de contrainte. Avantageusement, ladite au moins une jauge de contrainte est positionnée sur une plaque agencée de manière à se déformer en fonction de la position relative entre le système 2 et ledit corps de pompe 3. Préférentiellement, lesdits moyens 35 comprennent deux ou quatre jauges de contrainte.

Ladite pompe 1 comprend aussi des moyens de pilotage 36 du déplacement du corps de pompe 3 par rapport au système 2 en fonction de ladite grandeur déterminée.

Lesdits moyens de pilotage sont formés par une unité électronique et informatique de traitement et de calcul. Ladite unité peut être réalisée sous la forme d'un circuit électronique muni d'un microcontrôleur ou d'un microprocesseur associé à une mémoire de stockage de données. Ainsi, lorsque, dans la suite de la description, il est précisé que des moyens donnés sont configurés pour réaliser une opération donnée, cela signifie que le système électronique et informatique qui forme lesdits moyens, comprend des instructions informatiques permettant de réaliser ladite opération.

Lesdits moyens de pilotage 36 comprennent des moyens de définition 360 d'une valeur de consigne de ladite grandeur. Cette valeur de consigne correspond à l'effort de serrage souhaité du tube 5 entre le système 2 et le corps de pompe 3. Lesdits moyens de pilotage 36 comprennent aussi des moyens de régulation 361 configurés pour réguler le déplacement du corps de pompe 3 dans le sens d'un rapprochement ou d'un écartement du système d'application d'effort 2 en fonction de la valeur déterminée de ladite grandeur pour atteindre ladite valeur de consigne.

Ainsi, lesdits moyens de régulation 36 sont configurés pour acquérir par l'intermédiaire des moyens 35 de détermination, la valeur de ladite grandeur représentative de l'effort appliqué sur la paroi périphérique du tube 5, et pour comparer cette valeur acquise à la valeur de consigne mémorisée. En fonction du résultat de cette comparaison les moyens de pilotage 36 commandent le moteur 31 pour qu'il déplace le corps de pompe 3 par rapport au système 2 dans le sens d'un rapprochement ou d'un écartement jusqu'à atteindre une valeur proche de la consigne ou située dans une plage donnée par rapport à la valeur de consigne.

Lesdits moyens d'entrainement en déplacement des organes d'appui 7 comprennent un élément en boucle 6 qui relie entre eux les organes d'appui 7, et deux rouleaux 8 rotatifs, positionnés à l'intérieur et aux extrémités opposées dudit élément en boucle 6. Au moins l'un des rouleaux 8 est motorisé pour entrainer en déplacement l'élément en boucle 6 autour desdits rouleaux 8. Avantageusement, les organes d'appui 7 sont des galets.

L'élément en boucle 6 comprend une courroie de transmission agencée en boucle autour des rouleaux 8. L'un des rouleaux 8 est animé par un moteur 81 de sorte que ledit rouleau forme une roue menante apte à mener l'entrainement de la courroie autour desdits rouleaux. L'autre rouleau forme un support de guidage du déplacement de la courroie.

Les galets 7 sont montés solidaires en déplacement de la courroie 6 et font saillie de la face externe de ladite courroie pour permettre de presser le tube lors du déplacement desdits galets le long du tube.

Comme expliqué ci-dessus, l'élément en boucle comprend au moins une portion sensiblement droite qui s'étend sensiblement parallèlement à la surface plane 32 du corps de pompe 3, de sorte que les galets qui sont déplacés le long cette portion droite pressent le tube depuis une extrémité de cette portion vers l'autre extrémité.

Ladite pompe comprend un bâti 10 dans lequel sont logés le système 2 et le corps de pompe 3. Ledit corps de pompe 3 est monté mobile par rapport audit bâti 10.

Comme illustré plus particulièrement à la figure 3, ladite pompe 1 comprend aussi une membrane 9 qui, à l'état inséré du tube 5 entre le système 2 d'application d'effort et le corps de pompe 3, s'étend autour de la paroi périphérique dudit tube 5 pour former en coopération avec une paroi du bâti 10, opposée au fond de la membrane, une enveloppe de protection autour dudit tube 5.

Ladite membrane 9 est réalisée en un matériau souple étanche aux liquides.

Avantageusement, on peut prévoir que l'espace laissé libre entre le système 2 et le corps de pompe 3 soit situé en partie supérieure de la pompe pour pouvoir être aisément accessible par l'opérateur.

La présente invention n'est nullement limitée aux modes de réalisation décrits et représentés, mais l'homme du métier saura y apporter toute variante conforme à la portée des revendications annexées.

## Revendications

1. Pompe (1) péristaltique, par exemple pour machine de dialyse, comprenant, d'une part, un plateau, appelé corps de pompe (3), qui comprend une surface sensiblement plane (32) contre laquelle est destiné à être positionné un tube souple (5) de passage de fluide, et, d'autre part, un système (2) d'application d'effort comprenant une pluralité d'organes d'appui (7), tels que des galets, et des moyens d'entrainement en déplacement desdits d'organes d'appui (7) permettant de déplacer lesdits organes d'appui contre le tube pour le déformer contre ledit corps de pompe (3),
ledit corps de pompe (3) étant monté mobile par rapport au système (2) d'application d'effort entre une position écartée dudit système (2) d'application d'effort, et une position rapprochée dudit système (2) d'application d'effort, **caractérisée en ce que** ladite pompe (1) comprend des moyens (35) de détermination d'une grandeur représentative de l'effort appliqué sur le tube (5), à l'état positionné dudit tube (5) entre le système d'application d'effort (2) et le corps de pompe (3),
**en ce que** ladite pompe (1) comprend aussi des moyens de pilotage (36) du déplacement du corps de pompe (3) par rapport au système (2) en fonction de ladite grandeur déterminée,
et **en ce que** lesdits moyens de pilotage (36) comprennent des moyens de définition (360) d'une valeur de consigne de ladite grandeur, et des moyens de régulation (361) configurés pour réguler le déplacement du corps de pompe (3) dans le sens d'un rapprochement ou d'un écartement du système d'application d'effort (2) en fonction de la valeur déterminée de ladite grandeur pour atteindre ladite valeur de consigne.

2. Pompe (1) selon la revendication 1, **caractérisée en ce que** ledit corps de pompe (3) est monté mobile en translation suivant une direction transversale, de préférence orthogonale, à ladite surface plane (32) du corps de pompe (3).

3. Pompe (1) selon l'une des revendications précédentes, **caractérisée en ce que** ladite pompe (1) comprend des moyens moteurs (31) d'entrainement en déplacement dudit corps de pompe (3) entre ladite position rapprochée et ladite position écartée.

4. Pompe (1) selon l'une des revendications précédentes, **caractérisée en ce que** lesdits moyens (35) de détermination d'une grandeur représentative de l'effort appliqué sur le tube (5), comprennent au moins une jauge de contrainte, de préférence une pluralité de jauges de contrainte.

5. Pompe (1) selon l'une des revendications précédentes, **caractérisé en ce que** lesdits moyens d'entrainement en déplacement des organes d'appui (7) comprennent un élément en boucle (6) qui relie entre eux les organes d'appui (7), et deux rouleaux (8) rotatifs, positionnés à l'intérieur et aux extrémités opposées dudit élément en boucle (6), au moins l'un des rouleaux(8) étant motorisé pour entrainer en déplacement l'élément en boucle (6) autour desdits rouleaux (8).

6. Pompe (1) selon l'une des revendications précédentes, **caractérisé en ce que** ladite pompe comprend un bâti (10) dans lequel sont logés le système (2) et le corps de pompe (3),
et **en ce que** le corps de pompe (3) est monté mobile par rapport audit bâti (10).

7. Pompe (1) selon l'une des revendications précédentes, **caractérisée en ce que** ladite pompe comprend un bâti (10) dans lequel sont logés le système (2) et le corps de pompe (3),
et **en ce que** ladite pompe (1) comprend une membrane (9) qui, à l'état inséré du tube (5) entre le système (2) d'application d'effort et le corps de pompe (3), s'étend autour de la paroi périphérique dudit tube (5) pour former en coopération avec une paroi du bâti (10) une enveloppe de protection autour dudit tube (5).

8. Pompe (1) selon la revendication 7, **caractérisée en ce que** ladite membrane (9) est réalisée en un matériau souple étanche aux liquides.

## Patentansprüche

1. Peristaltische Pumpe (1), beispielsweise für Dialysemaschine, umfassend zum einen eine als Pumpenkörper (3) bezeichnete Platte, die eine etwa ebene Fläche (32) umfasst, welche für die Positionierung eines Fluiddurchgangsschlauchs (5) bestimmt ist, und zum anderen ein Kraftanwendungssystem (2), welches eine Vielzahl von Stützorganen (7) wie Rollen und Verlagerungsantriebsmittel der Stützorgane (7) umfasst, die erlauben, die Stützorgane gegen den Schlauch zu verlagern, um ihn gegen den Pumpenkörper (3) zu verformen,
wobei der Pumpenkörper (3) in Bezug zum Kraftanwendungssystem (2) zwischen einer vom Kraftanwendungssystem (2) beabstandeten Position und einer an das Kraftanwendungssystem (2) angenäherten Position beweglich montiert ist,
**dadurch gekennzeichnet, dass** die Pumpe (1) Bestimmungsmittel (35) einer für die auf den Schlauch (5) ausgeübten Kraft repräsentativen Größe im zwischen dem Kraftanwendungssystem (2) und dem Pumpenkörper (3) positionierten Zustand des Schlauchs (5) umfasst,
und dass die Pumpe (1) ebenfalls Steuermittel (36) der Verlagerung des Pumpenkörpers (3) in Bezug zum System (2) in Abhängigkeit von der bestimmten Größe umfasst,
und dass die Steuermittel (36) Definitionsmittel (360) eines Sollwerts der Größe und Einstellmittel (361) umfassen, die konfiguriert sind, um die Verlagerung des Pumpenkörpers (3) in die Richtung einer Annäherung oder einer Beabstandung des Kraftanwendungssystems (2) in Abhängigkeit von dem von der Größe bestimmten Wert einzustellen, um den Sollwert zu erreichen.

2. Pumpe (1) nach Anspruch 1, **dadurch gekennzeichnet, dass** der Pumpenkörper (3) in einer transversalen, vorzugsweise orthogonalen Richtung zur ebenen Fläche (32) des Pumpenkörpers (3) verschiebend beweglich montiert ist.

3. Pumpe (1) nach einem der vorangehenden Ansprüche, **dadurch gekennzeichnet, dass** die Pumpe (1) Antriebsmotormittel (31) für die Verlagerung des Pumpenkörpers (3) zwischen der angenäherten Position und der beabstandeten Position umfasst.

4. Pumpe (1) nach einem der vorangehenden Ansprüche, **dadurch gekennzeichnet, dass** die Bestimmungsmittel (35) einer Größe, die für die auf den Schlauch (5) ausgeübte Kraft repräsentativ ist, mindestens ein Dehnungsmessgerät, vorzugsweise eine Vielzahl von Dehnungsmessgeräten, umfassen.

5. Pumpe (1) nach einem der vorangehenden Ansprüche, **dadurch gekennzeichnet, dass** die Verlagerungsantriebsmittel der Stützorgane (7) ein Schleifenelement (6), das die Stützorgane (7) miteinander verbindet, und zwei rotierende Walzen (8), die innen und an den entgegengesetzten Enden des Schleifenelements (6) positioniert sind, umfassen, wobei mindestens eine der Walzen (8) motorisiert ist, um das Schleifenelement (6) um die Walzen (8) verlagernd anzutreiben.

6. Pumpe (1) nach einem der vorangehenden Ansprüche, **dadurch gekennzeichnet, dass** die Pumpe ein Gestell (10) umfasst, in dem das System (2) und der Pumpenkörper (3) untergebracht sind,
und dass der Pumpenkörper (3) in Bezug zum Gestell (10) beweglich montiert ist.

7. Pumpe (1) nach einem der vorangehenden Ansprüche, **dadurch gekennzeichnet, dass** die Pumpe ein Gestell (10) umfasst, in dem das System (2) und der Pumpenkörper (3) untergebracht sind,
und dass die Pumpe (1) eine Membran (9) umfasst, die sich im zwischen dem Kraftanwendungssystem (2) und dem Pumpenkörper (3) eingesetzten Zustand des Schlauchs (5) um die Umfangswand des Schlauchs (5) erstreckt, um im Zusammenwirken mit einer Wand des Gestells (10) eine Schutzhülle um den Schlauch (5) zu bilden.

8. Pumpe (1) nach Anspruch 7, **dadurch gekennzeichnet, dass** die Membran (9) aus einem elastischen flüssigkeitsdichten Material hergestellt ist.

## Claims

1. A peristaltic pump (1), for example for a dialysis machine, on the one hand comprising a plate, called pump body (3), that comprises a substantially planar surface (32) against which a flexible fluid passage tube (5) is intended to be positioned, and on the other hand, a system (2) for applying force comprising a plurality of bearing members (7), such as rollers, and means for driving the movement of said bearing members (7) making it possible to move said bearing members against the tube to deform it against said pump body (3);
said pump body (3) being mounted so as to be able to move relative to the force application system (2) between a position separated from said force application system (2), and a position close to said force application system (2),
**characterized in that** said pump (1) comprises means (35) for determining a property representative of the force applied on the tube (5), in the state of said tube (5) positioned between the force application system (2) and the pump body (3),
**in that** said pump (1) also comprises means (36) for controlling the movement of the pump body (3) relative to the system (2) as a function of said determined property, and **in that** said control means (36) comprise means (360) for defining a setpoint value of said property, and regulating means (361) configured to regulate the movement of the pump body (3) in a direction bringing the force application system (2) closer or further away based on the determined value of said property to achieve said setpoint value.

2. The pump (1) according to claim 1, **characterized in that** said pump body (3) is mounted translatably in a direction transverse, preferably orthogonal, to said planar surface (32) of the pump body (3).

3. The pump (1) according to one of the preceding claims, **characterized in that** said pump (1) comprises motor means (31) for driving the movement of said pump body (3) between said closed position and said separated position.

4. The pump (1) according to one of the preceding claims, **characterized in that** said means (35) for determining a property representative of the force applied on the tube (5) comprise at least one strain gauge, preferably a plurality of strain gauges.

5. The pump (1) according to one of the preceding claims, **characterized in that** said means for driving the movement of the bearing members (7) comprise a loop element (6) that connects the bearing members (7) to one another, and two rotating rolls (8), positioned inside and at opposite ends of said element (6), at least one of the rolls (8) being motorized to drive the movement of the loop element (6) around said rolls (8).

6. The pump (1) according to one of the preceding claims, **characterized in that** said pump comprises a frame (10) in which the system (2) and the pump body (3) are housed,
and **in that** the pump body (3) is mounted movably relative to said frame (10).

7. The pump (1) according to one of the preceding claims, **characterized in that** said pump comprises a frame (10) in which the system (2) and the pump body (3) are housed,
and **in that** said pump (1) comprises a membrane (9) which, in the state of the tube (5) inserted between the force application system (2) and the pump body (3), extends around the peripheral wall of said tube (5) to form, in cooperation with a wall of the frame (10), a protective housing around said tube (5).

8. The pump (1) according to claim 7, **characterized in that** said membrane (9) is made from a liquid-tight flexible material.
